# EUROPEAN PATENT SPECIFICATION

(11) **EP 2 827 911 B1**
(45) Date of publication and mention of the grant of the patent: **13.06.2018**
(21) Application number: 13720575.3
(22) Date of filing: 12.03.2013
(51) Int. Cl.: A61K 49/00, A61K 9/00

(54) **COLOURED SOLUTION IN PARTICULAR FOR USE IN SURGICAL METHODS FOR THE TREATMENT OF THE BODIES OF HUMANS OR ANIMALS**
GEFÄRBTE LÖSUNG, INSBESONDERE ZUR VERWENDUNG IN CHIRURGISCHEN VERFAHREN, ZUR BEHANDLUNG VON KÖRPERN VON MENSCHEN ODER TIEREN
SOLUTION COLORÉE EN PARTICULIER DESTINÉE À ÊTRE UTILISÉE DANS DES PROCÉDÉS CHIRURGICAUX POUR LE TRAITEMENT DES CORPS D'ÊTRES HUMAINS OU D'ANIMAUX

(30) Priority: 19.03.2012 IT VR20120049
(43) Date of publication of application: 28.01.2015
(73) Proprietor: AL.CHI.MI.A. S.r.l., 35020 Ponte San Nicolò (PD) (IT)
(72) Inventor: BECCARO, Mauro, 35010 Cadoneghe (Padova) (IT); BETTINI, Enrico, 30032 Fiesso D'Artico (Venezia) (IT); SIGNORI, Paolo, 37131 Verona (IT); GARCIA ARUMI, Jose, Barcelona (ES)
(74) Representative: Ponchiroli, Simone
(86) International application number: PCT/IB2013/051951
(87) International publication number: WO 2013/140300

(56) References cited:
- WO-A1-2009/139973
- WO-A2-01/44811
- WO-A2-2011/151079
- STANISLAO RIZZO ET AL: "Colored perfluorocarbon liquids as novel intraoperative tools", GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE, vol. 250, no. 5, 2 December 2011 (2011-12-02), pages 653-659, XP035046425, ISSN: 1435-702X, DOI: 10.1007/S00417-011-1874-7 cited in the application

## Description

### Technical field

This invention relates to a solution of a perfluorocarbon with a dye, in particular intended for use in surgical methods for the treatment of the bodies of humans or animals.

In particular, this invention was prepared with reference to the sector of ophthalmic surgery and even more particularly that of vitreoretinal surgery.

Therefore, hereinafter explicit reference will be made to said sector, without thereby excluding other applications.

### Background art

In surgeries involving the retina, for example for the treatment of retinal detachment or retinal alterations, the vitreous humour must be removed (vitrectomy). Generally, three small incisions are made in the sclera of the eye, which allow access to the back chamber of the eye, where the vitreous humour is located. Surgical instruments can be inserted into the eye through the holes. To prevent the eye from losing its shape due to the resulting loss of pressure and to prevent secondary lesions, as the vitreous humour is sucked out, a substitute substance is inserted into the back chamber in its place, keeping the internal pressure constant.

In order to be able to then proceed with correct reattachment of the retina using a laser, the retina must be kept spread out and stationary in its physiological position. That result is normally guaranteed by the substance used to substitute the vitreous humour which acts as a temporary tamponade.

For many years now the prior art has involved the use of liquid perfluorocarbons as temporary tamponades for smoothing out and stabilising the retina during surgical manipulations.

Chemically, perfluorocarbons are completely synthetic compounds which contain only fluorine and carbon atoms. The main features of this class of chemical compounds are the fact that they are extremely inert chemically, biologically and physiologically. They are also not soluble in water or in lipophilic liquids, with which they instead form emulsions.

For example, WO 03/079927 proposes the use of fluorinated alkanes.

However, the most widely used perfluorocarbons include perfluorodecalin, perfluorooctane (or n-perfluorooctane) and perfluorophenanthrene.

Thanks to their low viscosity, these liquids can be infused directly on the end of the optic nerve using standard cannulas. In fact, thanks to their high density they sink towards the rear retina and slowly fill the vitreous cavity from the bottom up.

After the surgery and the consequent stabilisation of the retina, the perfluorocarbons are removed and are substituted with the conventional gaseous and liquid endo-tamponades.

Complete removal of the perfluorocarbons during this procedure is essential. Any residues of perfluorocarbons in the eye, and in particular in the back chamber, may in fact subsequently cause significant problems for the patient. First, they may cover the retina like a thin film, causing vision problems. Second, they may cause collateral effects such as inflammatory reactions.

However, the perfluorocarbons are transparent and colourless, which makes their complete removal extremely difficult.

To overcome that disadvantage, the idea of creating coloured yet still transparent tamponade liquids was developed. The aim was to make them easily identifiable by the surgeon (even small residual amounts).

However, due to the extreme inertness of perfluorocarbons, so far no dye substances have been identified which are able to colour them at least in a way that is stable and safe for patient health, as acknowledged in both patent application WO 2011/151079 and in the article by S. Rizzo, C. Belting, F. Genovesi-Ebert, N. Hagedorn, "Colored perfluorocarbon liquids as novel intraoperative tools", Graefes Arch Clin Exp Ophthalmol on line, 2 December 2011 (DOI 10.1007/s00417-011-1874-7).

In an attempt to overcome that problem, both of the documents just referred to in fact proposed an alternative solution for making coloured tamponade liquids, which involves mixing a colourless perfluorocarbon (completely fluorinated alkane) with a coloured liquid consisting of a solution in which a dye (in itself not soluble directly in the colourless perfluorocarbon) is dissolved in a semi-fluorinated alkane. In particular, whilst the above-mentioned article describes the use of only anthraquinone blue as a dye, the patent application describes preferably the use of Sudan-class dyes.

Although the preparations produced in this way may on one hand have a density suitable for allowing their use as tamponades, and on the other hand a colour suitable for making them visible, it is still a solution that is not without disadvantages.

First, preparations of this type are mixtures of two substances rather than solutions, with the risk that even inside the eye they may separate into the two phases. It is easy to understand how the separation of the colourless perfluorocarbon would cause the same disadvantages as the tamponades commonly used today.

Second, production of these preparations is relatively complex because it requires first colouring of the semi-fluorinated alkanes and then their homogeneous mixing with the fluorinated alkanes.

Moreover, the fact that, as also indicated in the above-mentioned article by Rizzo et al., the semi-fluorinated alkanes have a density that is too low, puts limits on the maximum quantity of semi-fluorinated alkanes which can be used in the mixture.

A further major disadvantage is highlighted in the article by Rizzo et al., where it indicates that the preparation tends to release the dye when it is put in contact with silicone oil (until it even loses it completely in a relatively short time).

That means not only that the silicone oil may be contaminated when the two substances make contact during the surgery, but also that there is a real risk, if the preparation remains in the eye too long, that the dye may migrate (for the same reasons for which it migrates into the silicone oil) into the fatty parts of the tissues with which it makes contact. In fact, they are lipophilic dyes which can bind to the tissue to some degree, making it difficult to remove them.

### Disclosure of the invention

In this context the technical purpose which forms the basis of this invention is to provide a coloured solution which overcomes the above-mentioned disadvantages.

In particular, the technical purpose of this invention is to provide a coloured solution which is biocompatible and which can be used as a retinal tamponade.

It is also the technical purpose of this invention to provide a coloured solution which is stable with respect to laser treatments of the type used for vitreoretinal surgery.

Furthermore, it is the technical purpose of this invention to provide a coloured solution which has a reduced risk of release of the dye compared with the prior art solutions.

The technical purpose specified and the aims indicated are substantially achieved by a coloured solution as described in the appended claims.

Further features and the advantages of this invention are more apparent in the detailed description below of several preferred, non-limiting embodiments of a coloured solution.

### Detailed description of preferred embodiments of the invention

In general, according to a first aspect of this invention, the coloured solution comprises on one hand at least one perfluorocarbon which has the function of a solvent, and on the other hand a dye directly dissolved into the perfluorocarbon and selected from the family of fluorinated derivatives of phthalocyanines obtained by substituting one or more of the hydrogen H atoms of the phthalocyanine linked to the benzene rings with a fluorine atom or a fluorinated group, and it is intended for use in surgical methods for the treatment of the bodies of humans or animals.

According to a second aspect of this invention, the coloured solution comprises on one hand at least one perfluorocarbon selected from perfluorooctane (CAS No. 307-34-6), perfluorodecalin (CAS No. 306-94-5) and perfluorophenanthrene (CAS No. 306-91-2) and which has the function of a solvent, and on the other hand a dye directly dissolved into the perfluorocarbon and selected from the family of fluorinated derivatives of phthalocyanines obtained by substituting one or more of the hydrogen H atoms of the phthalocyanine linked to the benzene rings with a fluorine atom or a fluorinated group. This solution is intended for use both in surgical methods and for any other application for which it may be useful.

It should be noticed that the terms perfluorooctane, perfluorodecalin and perfluorophenanthrene in the context of this description and in the appended claims also include all synonyms commonly used which have the same CAS number.

According to all aspects of this invention, the dye is preferably selected from the family of fluorinated derivatives of metal phthalocyanines obtained by substituting one or more of the hydrogen H atoms of the phthalocyanine linked to the benzene rings with a fluorine atom or a fluorinated group.

Since the subject matter of this invention is a solution, it does not cover any mixtures of a perfluorocarbon and a fluorinated derivative of phthalocyanine of the type indicated above, which do not constitute a solution.

Therefore, the fluorinated derivatives of the phthalocyanines (metal or not) used in the context of this invention, that is to say, those obtained by substituting one or more of the hydrogen H atoms linked to the benzene rings with a fluorine atom or a fluorinated group, are defined by a formula: 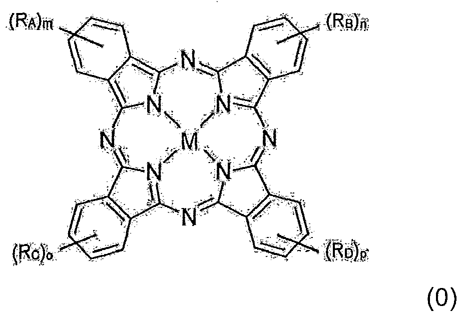 where:
- M may be absent or may be an ion or an atom or a group;
- m, n, o and p are, independently of each other, an integer between 0 and 4, at least one of them not being zero;
- R_{A}, R_{B}, R_{C} and R_{D} are, independently of each other, either a fluorine atom or a generic fluorinated group, advantageously perfluorinated, linked to one of the four external carbon atoms of the benzene ring to which it is connected. Moreover, for each benzene ring each R_{A}, R_{B}, R_{C} and R_{D} present in the formula (that is to say, if the related subscript item m, n, o and p is greater than 1) may be an atom or a group which is the same as or different to the others of the same benzene ring or of the other benzene rings.

In other words, the dye may have, at each of the four benzene rings of the phthalocyanine, one or more fluorine atoms or fluorinated groups, up to a maximum of four, which may either be the same as each other or different.

Each of said atoms or groups is linked to one of the four carbon atoms of the benzene ring in place of the hydrogen H atom which would normally be present in an unmodified phthalocyanine.

Even more preferably, the dye belongs to the family of metalpoly(fluoroalkyl)phthalocyanines and advantageously to that of metalpoly(perfluoroalkyl)phthalocyanines. It should be noticed that these are dyes which are easily found on the market and whose synthesis has been known to experts in the field for many years, as indicated for example in patents US 3,281,426 and US 7,473,782. Consequently, they are not described in further detail herein.

Therefore, advantageously, in the preferred embodiment of this invention, the dye has a formula: 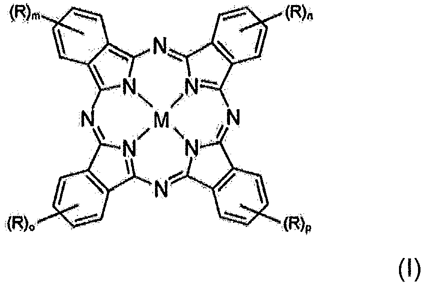 where:
- M is at least one metal ion or one metal atom (which may be linked also to one or more atoms of other elements);
- m, n, o and p are, independently of each other, an integer between 0 and 4, at least one of them not being zero;
- each R, independently of the others present, represents a generic fluoroalkyl group, advantageously perfluoroalkyl, linked to one of the four external carbon atoms of the benzene ring to which it is connected.

Moreover, for each benzene ring each R present in the formula (that is to say, if the related subscript item m, n, o and p is greater than 1) may represent a different fluoroalkyl or perfluoroalkyl group.

In other words, the dye may have, at each of the four benzene rings of the phthalocyanine, one or more fluoroalkyl or perfluoroalkyl groups, up to a maximum of four, which may either be the same as each other or different.

Each of said groups is linked to one of the four carbon atoms of the benzene ring in place of the hydrogen atom which would normally be present in an unmodified phthalocyanine.

Advantageously, each fluoroalkyl or perfluoroalkyl group R comprises a number of carbon atoms which is between 3 and 16. In particular, each fluoroalkyl or perfluoroalkyl group R may be either linear and have the formula CF₃(CF₂)_{q}-, where q is an integer between 2 and 15, or branched, or cyclical.

The metal part M of the molecule advantageously consists of an atom of an element selected from copper, cobalt and zinc, and is preferably copper.

In this case the formula (I) becomes respectively:

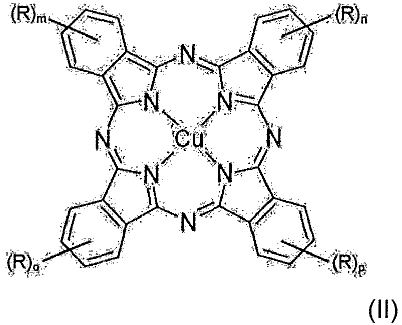

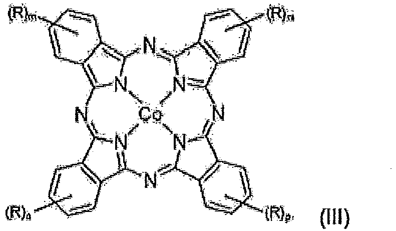

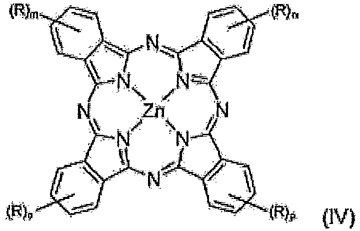

However, in a preferred embodiment the dye has the formula:

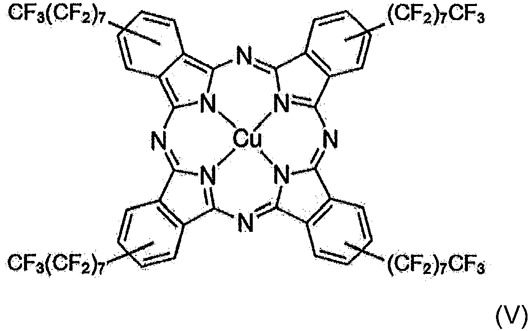

It should be noticed that in this dye each perfluoroalkyl group -(CF₂)₇CF₃ may be linked to any one of the external carbon atoms of the benzene ring, in place of one of the four hydrogen H atoms present in the unmodified phthalocyanine.

However, in general, since the fluorinated derivatives of phthalocyanines considered by this invention may adopt various colours, the choice of colour may be made on each occasion depending on requirements for use.

With reference to the coloured solution as a whole, preferably the weight ratio of solute to solvent is between 0.0008% and 0.01%, and preferably between 0.005% and 0.01%.

However, in general the quantity of dye used will be selected in such a way that the solution obtained with it has a colour which is sufficient but not excessive for the purpose for which it will be used.

In fact, advantageously, despite having a colour which makes it clearly visible, the coloured solution is substantially transparent. That means that during use the coloured solution must allow what is behind it to be seen. In particular, when used in a vitreoretinal surgical method, it must allow the structures of the eye, such as the retina, behind it to be seen.

For example, in a preferred composition of the coloured solution according to this invention, the solvent is selected from the following compounds: perfluorooctane with the formula 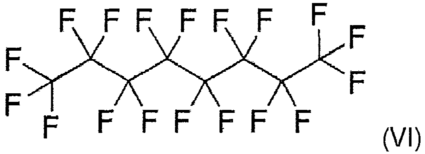 perfluorodecalin with the formula 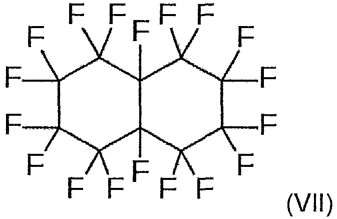 and perfluorophenanthrene with the formula 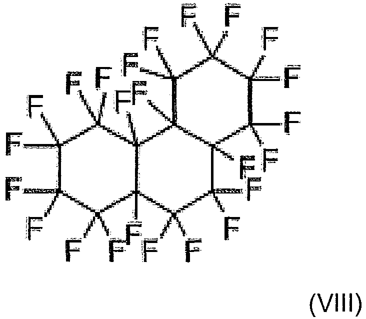 whilst the dye is defined by the formula (V) and is present in a weight ratio of 0.008 % to the solvent. In particular, with the solvent consisting of perfluorooctane (VI), the solution has an absorbance equal to 1,403 at a wavelength of 580 nm. Said solution also has a density of 1,756 kg/I at a temperature of 20°C (substantially equal to that of perfluorooctane alone which, at 20°C, is equal to 1,755).

Regarding the physical properties of the coloured solution (such as density and viscosity), it should also be noticed that, also thanks to the small quantities of dye used in the preferred embodiments, advantageously they do not significantly deviate from those of the solvent alone.

In any case, the coloured solution is liquid at least in the temperature range between 0°C and 50°C.

With reference to the surgical methods for which the coloured solution according to this invention is intended, in particular it is intended to be used in a vitreoretinal surgical method, and even more particularly as a retinal tamponade. In the latter case, during the vitreoretinal surgery the coloured solution according to this invention is injected into the back chamber and acts as a temporary tamponade for spreading out and stabilising the retina. However, advantageously, the coloured solution is removed when a predetermined period of time has elapsed after it was injected. During removal the dye present in the coloured solution provides a clear distinction between the coloured solution itself and the structures of the eye. Thanks to said distinction, it is easier to completely remove the coloured solution from the back chamber, and from the eye in general if there were any leaks during the surgery.

A second possible use of the coloured solution according to this invention is for identifying any residual vitreous humour during removal of the vitreous humour. In this case, the coloured solution is injected into the back chamber when the surgeon believes that he has removed all of the vitreous humour. In fact, thanks to the high density of the perfluorocarbons present in the solution, any residues of vitreous humour float on the coloured solution. At that point, the colour contrast obtained between any residues of vitreous humour and the coloured solution makes the former more visible.

Furthermore, the use of the coloured solution according to this invention makes the interface between the coloured solution and silicone oil clearly visible during the step of substituting the coloured solution with silicone oil (again in vitreoretinal surgeries).

An important aspect of the coloured solutions according to this invention, and in particular of those which use perfluorooctane, perfluorodecalin and perfluorophenanthrene as a solvent, is the fact that they have optimum biocompatibility and a substantial absence of release of the dye used.

Tests carried out by the Applicant demonstrated their stability even when subjected to laser treatment. In particular, the Applicant tested a sample of blue perfluorooctane in which the dye is defined by the formula (V), treating it in the following conditions:
- instrument used: endolaser photocoagulation with a laser diode made and marketed by the company Iridex, California;
- power used: between 400 and 600 mw;
- laser pulse duration: 9000 ms.

Before and after said treatment two NMR analyses were carried out which demonstrated that the laser treatment did not modify in any way the product tested.

It should be noticed that the test conditions selected are much more critical than those normally used in surgeries, where, generally speaking:
- the power is between 150 and 250 mw;
- the duration is between 200 and 500 ms.

It should also be noticed that the context of this invention also covers any concentrated solutions intended to be diluted by adding solvent before they are actually used.

Finally, both the concentrated solutions and the more dilute ones may also be used with the sole aim of facilitating the removal of a conventional fluorinated endo-tamponade at the end of the surgery. In fact, in that case, after using a conventional transparent perfluorinated liquid, the surgeon can inject the coloured solution at the end of the surgery. In fact, the coloured solution quickly mixes with the transparent perfluorinated liquid, colouring it and so making it easier to see.

This invention therefore brings important advantages.

First, thanks to this invention it was possible to prepare coloured solutions which are biocompatible, which can be used as retinal tamponades and which are stable even after laser treatment.

In particular, it was possible to prepare coloured solutions which use as a solvent only perfluorinated substances, unlike the prior art.

Moreover, the coloured solutions according to this invention have practically no risk of releasing the dye, unlike the prior art solutions.

Finally, it should be noticed that this invention is relatively easy to produce and that even the cost linked to implementing the invention is not very high.

The invention described above may be modified and adapted in several ways without thereby departing from the scope of the inventive concept.

Moreover, all details of the invention may be substituted with other technically equivalent elements and the materials used, as well as the shapes and dimensions of the various components, may vary according to requirements.

## Claims

1. A coloured solution comprising at least one perfluorocarbon as a solvent and a dye dissolved in the perfluorocarbon and selected from the family of fluorinated derivatives of phthalocyanines obtained by substituting one or more of the hydrogen H atoms linked to the benzene rings with a fluorine atom or a fluorinated group, for use in surgical methods for the treatment of the bodies of humans or animals, **wherein the dye is directly dissolved into the perfluorocarbon.**

2. The coloured solution according to the preceding claim, for use in a surgical method for vitreoretinal surgery.

3. The coloured solution according to claim 2, for use as a retinal tamponade.

4. The coloured solution for use according to claim 3, wherein during the surgical method the coloured solution is injected into the back chamber and acts as a temporary tamponade for spreading out and stabilising the retina.

5. The coloured solution for use according to claim 4, wherein the coloured solution is removed when a predetermined period of time has elapsed after it was injected, and wherein the dye present in the coloured solution provides a clear distinction between the coloured solution itself and the structures of the eye, said distinction facilitating complete removal of the coloured solution.

6. A coloured solution comprising at least one perfluorocarbon selected from perfluorooctane, perfluorodecalin and perfluorophenanthrene as a solvent and a dye dissolved in the perfluorocarbon and selected from the family of fluorinated derivatives of phthalocyanines obtained by substituting one or more of the hydrogen H atoms linked to the benzene rings with a fluorine atom or a fluorinated groups, **wherein the dye is directly dissolved into the perfluorocarbon.**

7. The coloured solution according to the preceding claim, wherein the dye is selected from the family of metalpoly(fluoroalkyl)phthalocyanines.

8. The coloured solution according to the preceding claim, wherein the dye is selected from the family of metalpoly(perfluoroalkyl)phthalocyanines.

9. The coloured solution according to claim 7, wherein the dye has the formula: 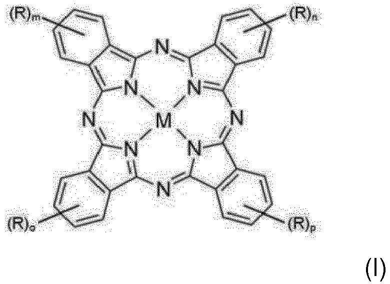 where:
- M is at least one metal ion or one metal atom (which may be linked also to one or more atoms of other elements);
- m, n, o and p are, independently of each other, an integer between 0 and 4, at least one of them not being zero;
- each R independently of the others present represents a generic fluoroalkyl group;
each fluoroalkyl group R present may be the same as, or different from, one or more of the other fluoroalkyl groups present.

10. The coloured solution according to claims 8 and 9, wherein each R independently represents a generic perfluoroalkyl group.

11. The coloured solution according to the preceding claim, wherein perfluoroalkyl group R comprises a number of carbon atoms which is between 3 and 16.

12. The coloured solution according to the preceding claim, wherein each perfluoroalkyl group R is linear and has the formula CF₃(CF₂)_{q}-, where q is an integer between 2 and 15.

13. The coloured solution according to any one of the claims from 8 to 12, where M represents a copper, cobalt or zinc atom.

14. The coloured solution according to any one of the claims from 6 to 13, wherein the weight ratio of solute to solvent is between 0.0008% and 0.1%.

15. The coloured solution according to any one of the claims from 6 to 13, wherein the weight ratio of solute to solvent is between 0.005% and 0.01%.

16. The coloured solution according to any one of the claims from 6 to 15, **characterised in that** it is also substantially transparent.

17. The coloured solution according to any one of the claims from 6 to 16, **characterised in that** it is also liquid at least in the temperature range between 0°C and 50°C.

18. The coloured solution according to any one of the claims from 6 to 17, **characterised in that** it is also for use in surgical methods for the treatment of the bodies of humans or animals in accordance with any one of the claims from 1 to 5.

## Patentansprüche

1. Eine gefärbte Lösung, mindestens einen perfluorierten Kohlenwasserstoff als Lösungsmittel und einen Farbstoff, der im perfluorierten Kohlenwasserstoff aufgelöst ist und aus der Familie der fluorierten Derivate von Phthalocyaninen gewählt wurde, erhalten durch die Ersetzung eines oder mehrerer der mit den Benzolringen verbundenen Wasserstoff-H-Atomen durch ein Fluoratom oder eine fluorierte Gruppe, zur Verwendung in chirurgischen Verfahren zur Behandlung der Körper von Menschen oder Tieren, wobei der Farbstoff direkt im perfluorierten Kohlenwasserstoff aufgelöst ist.

2. Die gefärbte Lösung nach dem vorherigen Patentanspruch zur Verwendung in einem chirurgischen Verfahren zur vitreoretinalen Chirurgie.

3. Die gefärbte Lösung nach dem Patentanspruch 2, zur Verwendung als Netzhauttamponade.

4. Die gefärbte Lösung zur Verwendung
nach dem Patentanspruch 3, wobei während des chirurgischen Verfahrens die gefärbte Lösung in die hintere Augenkammer injiziert wird und als vorübergehende Tamponade dient, um sich auszudehnen und die Netzhaut zu stabilisieren.

5. Die gefärbte Lösung zur Verwendung
nach dem Patentanspruch 4, wobei die gefärbte Lösung entfernt wird, wenn eine festgelegte Zeitspanne nach ihrer Injektion vergangen ist, und wobei der Farbstoff, der in der gefärbten Lösung enthalten ist, eine deutliche Unterscheidung zwischen der gefärbten Lösung selbst und den Strukturen des Auges bietet, besagte Unterscheidung erleichtert dabei die vollständige Entfernung der gefärbten Lösung.

6. Eine gefärbte Lösung, welche mindestens einen perfluorierten, aus Perfluoroctan, Perfluorodecalin und Perfluorphenanthren gewählten, Kohlenwasserstoff als Lösungsmittel enthält und einen Farbstoff, der im perfluorierten Kohlenwasserstoff aufgelöst ist und aus der Familie der fluorierten Derivate von Phthalocyaninen gewählt wurde, erhalten durch die Ersetzung eines oder mehrerer der Wasserstoff-H-Atome, die mit den Benzolringen verbunden sind, durch ein Fluoratom oder eine fluorierte Gruppe, wobei der Farbstoff direkt im perfluorierten Kohlenwasserstoff aufgelöst ist.

7. Die gefärbte Lösung nach dem vorherigen Patentanspruch, wobei der Farbstoff aus der Familie der Metall Poly-(Fluoroalkyl)-Phthalocyanine gewählt ist.

8. Die gefärbte Lösung nach dem vorherigen Patentanspruch, wobei der Farbstoff aus der Familie der Metall Poly-(Perfluoroalkyl)-Phthalocyanine gewählt ist.

9. Die gefärbte Lösung nach dem Patentanspruch 7, wobei der Farbstoff folgende Formel hat: 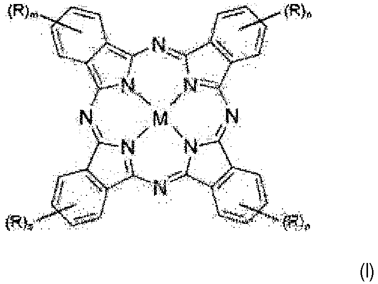 Dabei:
- ist M mindestens ein Metallion oder ein Metallatom (das ebenfalls mit einem oder mehreren Atomen anderer Elemente verbunden sein kann);
- sind m, n, o und p unabhängig voneinander ein Integer zwischen 0 und 4, wobei mindestens eines davon nicht Null ist,
- stellt jedes R unabhängig von anderen vorhandenen eine allgemeine Fluoroalkylgruppe dar;
kann jede vorhandene Fluoroalkylgruppe R die gleiche sein wie eine oder mehrere der anderen vorhandenen Fluoroalkylgruppen oder davon abweichen.

10. Die gefärbte Lösung nach den Patentansprüchen 8 und 9, wobei jedes R unabhängig eine allgemeine Perfluoroalkylgruppe darstellt.

11. Die gefärbte Lösung nach dem vorherigen Patentanspruch, wobei die Perfluoroalkylgruppe R eine Anzahl von Kohlenstoffatomen umfasst, die zwischen 3 und 16 liegt.

12. Die gefärbte Lösung nach dem vorherigen Patentanspruch, wobei jede Perfluoroalkylgruppe R linear ist und die Formel CF₃(CF₂)_{q}- hat, wobei jedes q ein Integer zwischen 2 und 15 ist.

13. Die gefärbte Lösung nach jedem der Patentansprüche von 8 bis 12, wobei M ein Kupfer-, Kobalt- oder Zinkatom darstellt.

14. Die gefärbte Lösung nach jedem der Patentansprüche von 6 bis 13, wobei das Gewichtsverhältnis zwischen dem gelösten Stoff und dem Lösungsmittel zwischen 0,0008% und 0,1% beträgt.

15. Die gefärbte Lösung nach jedem der Patentansprüche von 6 bis 13, wobei das Gewichtsverhältnis zwischen dem gelösten Stoff und dem Lösungsmittel zwischen 0,005% und 0,01% beträgt.

16. Die gefärbte Lösung nach jedem der Patentansprüche von 6 bis 15, **gekennzeichnet dadurch, dass** sie außerdem im Wesentlichen transparent ist.

17. Die gefärbte Lösung nach jedem der Patentansprüche von 6 bis 16, **gekennzeichnet dadurch, dass** sie auch flüssig ist, zumindest im Temperaturbereich zwischen 0° C und 50° C.

18. Die gefärbte Lösung nach jedem der Patentansprüche von 6 bis 17, **gekennzeichnet dadurch, dass** sie auch zur Verwendung bei chirurgischen Verfahren zur Behandlung der Körper von Menschen oder Tieren in Übereinstimmung mit jedem der vorherigen Patentansprüche von 1 bis 5 dient.

## Revendications

1. Une solution colorée comprenant au moins un perfluorocarbone en tant que solvant et un colorant dissous dans le perfluorocarbone et choisi dans la famille des dérivés fluorés des phthalocyanines obtenus par substitution d'un ou de plusieurs des atomes d'hydrogène H liés aux anneaux benzéniques avec un atome de fluor ou un groupe fluoré, destinée à être utilisée dans des procédés chirurgicaux pour le traitement des corps d'êtres humains ou d'animaux, où le colorant est directement dissous dans le perfluorocarbone.

2. La solution colorée selon la revendication précédente, destinée à être utilisée dans un procédé chirurgical de chirurgie vitréo-rétinienne.

3. La solution colorée selon la revendication 2, destinée à être utilisée en tant que tamponnement rétinien.

4. La solution colorée à utiliser selon la revendication 3, où, pendant le procédé chirurgical, la solution colorée est injectée dans la chambre arrière et agit en tant que tamponnement temporaire pour élargir et stabiliser la rétine.

5. La solution colorée à utiliser selon la revendication 4, où la solution colorée est éliminée quand une période de temps déterminée s'est écoulée après son injection, et où le colorant présent dans la solution colorée fournit une distinction claire entre la solution colorée elle-même et les structures de l'oeil, ladite distinction facilitant l'élimination complète de la solution colorée.

6. Une solution colorée comprenant au moins un perfluorocarbone choisi parmi le perfluorooctane, la perfluorodécaline et le perfluorophénanthrène en tant que solvant et un colorant dissous dans le perfluorocarbone et choisi dans la famille des dérivés fluorés des phthalocyanines obtenus par substitution d'un ou de plusieurs des atomes d'hydrogène H liés aux anneaux benzéniques avec un atome de fluor ou un groupe fluoré, où le colorant est directement dissous dans le perfluorocarbone.

7. La solution colorée selon la revendication précédente, où le colorant est choisi dans la famille des metalpoly(fluoroalkyle)phthalocyanines.

8. La solution colorée selon la revendication précédente, où le colorant est choisi dans la famille des metalpoly(perfluoroalkyle)phthalocyanines.

9. La solution colorée selon la revendication 7, où le colorant a la formule : 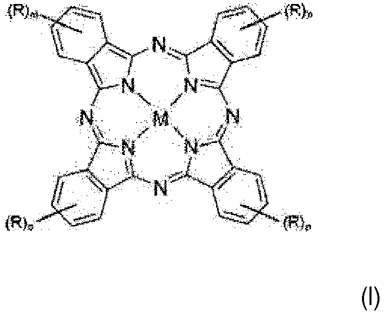 où :
- M représente au moins un ion métallique ou un atome de métal (qui peut également être lié à un ou plusieurs atomes d'autres éléments) ;
- m, n, o et p représentent, indépendamment l'un de l'autre, un nombre entier compris entre 0 et 4, au moins l'un d'eux n'étant pas zéro ;
- chaque R indépendamment des autres présents représente un groupe fluoroalkyle générique ;
chaque groupe fluoroalkyle R présent peut être le même que, ou différent de, un ou plusieurs autres groupes fluoroalkyles présents.

10. La solution colorée selon les revendications 8 et 9, où chaque R représente indépendamment un groupe perfluoroalkyle générique.

11. La solution colorée selon la revendication précédente, où le groupe perfluoroalkyle R comprend un nombre d'atomes de carbone qui est compris entre 3 et 16.

12. La solution colorée selon la revendication précédente, où chaque groupe perfluoroalkyle R est linéaire et a la formule CF₃(CF₂)_{q}-, où q est un nombre entier compris entre 2 et 15.

13. La solution colorée selon l'une quelconque des revendications de 8 à 12, où M représente un atome de cuivre, de cobalt ou de zinc.

14. La solution colorée selon l'une quelconque des revendications de 6 à 13, où le rapport en poids entre soluté et solvant est compris entre 0,0008 % et 0,1 %.

15. La solution colorée selon l'une quelconque des revendications de 6 à 13, où le rapport en poids entre soluté et solvant est compris entre 0,005 % et 0,01 %.

16. La solution colorée selon l'une quelconque des revendications de 6 à 15, **caractérisée en ce qu'**elle est aussi essentiellement transparente.

17. La solution colorée selon l'une quelconque des revendications de 6 à 16, **caractérisée en ce qu'**elle est aussi liquide au moins dans la plage de température comprise entre 0°C et 50°C.

18. La solution colorée selon l'une quelconque des revendications de 6 à 17, **caractérisée en ce qu'**elle est aussi destinée à être utilisée dans des procédés chirurgicaux pour le traitement des corps d'êtres humains ou d'animaux selon l'une quelconque des revendications de 1 à 5.
